(19) Europäisches Patentamt
European Patent Office
Office européen des brevets

(11) Veröffentlichungsnummer: **0 406 700 B1**

# (12) EUROPÄISCHE PATENTSCHRIFT

(45) Veröffentlichungstag der Patentschrift: **13.04.94**

(51) Int. Cl.$^5$: **C07D 275/04**, A01N 43/80

(21) Anmeldenummer: **90112326.5**

(22) Anmeldetag: **28.06.90**

## (54) 3-Anilino-benzisothiazole und diese enthaltende Fungizide.

(30) Priorität: **05.07.89 DE 3922088**

(43) Veröffentlichungstag der Anmeldung:
**09.01.91 Patentblatt 91/02**

(45) Bekanntmachung des Hinweises auf die Patenterteilung:
**13.04.94 Patentblatt 94/15**

(84) Benannte Vertragsstaaten:
**AT BE CH DE DK ES FR GB GR IT LI NL SE**

(56) Entgegenhaltungen:
**EP-A- 0 133 418**
**EP-A- 0 244 705**
**DE-A- 3 343 091**
**DE-A- 3 544 436**

(73) Patentinhaber: **BASF Aktiengesellschaft**
**Carl-Bosch-Strasse 38**
**D-67063 Ludwigshafen(DE)**

(72) Erfinder: **Schubert, Juergen, Dr.**
**Am Oberen Luisenpark 2**
**D-6800 Mannheim 1(DE)**
Erfinder: **Wild, Jochen, Dr.**
**An der Marlach 7**
**D-6705 Deidesheim(DE)**
Erfinder: **Hagen, Helmut, Dr.**
**Max-Slevogt-Strasse 17e**
**D-6710 Frakenthal(DE)**
Erfinder: **Nilz, Gerhard, Dr.**
**Haardtstrasse 13**
**D-6701 Dannstadt-Schauernheim(DE)**
Erfinder: **Sauter, Hubert, Dr.**
**Neckarpromenade 20**
**D-6800 Mannheim(DE)**
Erfinder: **Ammerman, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 406 700 B1

## Beschreibung

Die vorliegende Erfindung betrifft neue, wertvolle 3-Anilino-benzisothiazole, Verfahren zu ihrer Herstellung, diese Verbindungen enthaltende fungizide Mittel und ihre Verwendung als Fungizide.

Es ist bekannt, daß 2-Anilino-Benzthiazole, zum Beispiel das 2-(2,6-Dinitro-3-chlor-4-trifluormethylanilino)-6-methoxybenzthiazol oder das 2-(2,6-Dinitro-3-chlor-4-trifluormethylanilino)-6-nitrobenzthiazol (EP 244 705), eine gute fungizide Wirkung zeigen. Die Wirkung ist jedoch bei niedrigen Aufwandmengen und Anwendungskonzentrationen nicht immer befriedigend.

Überraschend wurde nun gefunden, daß 3-Anilino-benzisothiazole der Formel I

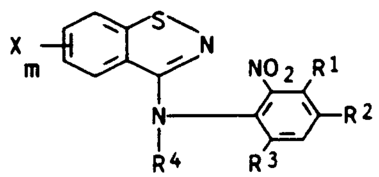

I,

in welcher

X Wasserstoff, $NO_2$, CN, Halogen, $SO_2$-$C_1$-$C_4$-Alkyl, $C_1$-$C_4$-Alkyl, $C_3$-$C_6$-Cycloalkyl, $C_1$-$C_4$-Halogenalkyl, $C_1$-$C_4$-Alkoxy, $C_1$-$C_4$-Halogenalkoxy $C_1$-$C_4$-Alkylthio, gegebenenfalls im Phenylrest durch $C_1$-$C_4$-Alkyl, Halogen, Nitro substituiertes Phenoxy oder Phenylthio bedeutet,

m eine ganze Zahl von 1 bis 4 ist, wobei die einzelnen Reste gleich oder verschieden sind, wenn m größer als 1 ist,

$R^1$ Wasserstoff, Halogen, $C_1$-$C_4$-Alkoxy, $C_1$-$C_4$-Halogenalkyl, $C_1$-$C_4$-Alkylthio, $C_1$-$C_4$-Halogenalkoxy, gegebenenfalls im Phenylrest durch $C_1$-$C_4$-Alkyl, Halogen, Nitro substituiertes Phenoxy, Phenylthio, Benzyloxy oder Benzylthio,

$R^2$, $R^3$ unabhängig voneinander Wasserstoff, $NO_2$, Halogen, CN, $C_1$-$C_6$-Alkyl, $SO_2$-$C_1$-$C_4$-Alkyl, $C_1$-$C_6$-Halogenalkyl, $SO_2NR^5R^6$, $C_1$-$C_4$-Halogenalkoxy, $COOR^5$, $CONR^5R^6$,

$R^4$ Wasserstoff, $COOR^7$, $CONR^5R^6$, CHO, $COR^7$, $SO_2R^7$,

$R^5$, $R^6$ unabhängig voneinander Wasserstoff, $C_1$-$C_4$-Alkyl,

$R^7$ $C_1$-$C_4$-Alkyl, $C_1$-$C_4$-Halogenalkyl, Benzyl, gegebenenfalls durch $C_1$-$C_4$-Alkyl, Halogen, Nitro substituiertes Phenyl oder Naphthyl bedeutet

und ihre pflanzenverträglichen Salze eine sehr gute fungizide Wirkung bei ausgezeichneter Verträglichkeit bei Pflanzen haben.

Unter dem Begriff Alkyl selbst oder als Bestandteil eines anderen Substituenten, wie Halogenalkyl, Alkoxy, Alkylthio, Halogenalkoxy sind je nach Zahl der angegebenen Kohlenstoffatome die folgenden geradkettigen oder verzweigten Gruppen zu verstehen: insbesondere $C_1$-$C_4$-Alkyl, Methyl, Ethyl, n-Propyl, iso-Propyl, n-Butyl, sec.-Butyl, iso-Butyl, tert.-Butyl, n-Pentyl, n-Hexyl. Die Vorsilbe Halogen in der Bezeichnung eines Substituenten bedeutet hier und im folgenden, daß dieser Substituent einfach bis mehrfach auftreten kann. Halogen steht stellvertretend für F, Cl, Br oder J. Halogenalkyl steht somit für einen einfach bis perhalogenierten Alkylrest, wie $CH_2Cl$, $CHCl_2$, $CCl_3$, $CH_2F$, $CHF_2$, $CF_3$, $CH_2Br$, CHBrCl, $CF_2Cl$, $C_2Cl_5$, $C_2F_5$, $CHF_2$, $CF_2$-$CHF_2$, $CH_2CH_2Cl$, $CH_2CH_2Br$, $C_3F_7$, $C_4F_9$.

Cycloalkyl steht für Cyclopropyl, Cyclobutyl, Cyclopentyl und Cyclohexyl, wobei die Ringe ein- bis zweifach methylsubstituiert sein können, wie z.B. 1-Methylcyclopropyl, 2-Methylcyclopropyl, 2,2-Dimethylcyclopropyl, 1-Methylcyclopentyl, 1-Methylcyclohexyl.

Gegebenenfalls substituiertes Phenyl oder Naphthyl steht, z.B. für Phenyl, $C_1$-$C_4$-Alkyl-, Phenyl, 2-, 3-, 4-Tolyl, Halogenphenyl, 4-Brom-phenyl, 4-Chlor-phenyl, 4-Fluor-phenyl, Nitrophenyl, 2-Nitrophenyl, 4-Nitrophenyl, 1- und 2-Naphthyl.

Gegebenenfalls substituiertes Phenoxy, Phenylthio, Benzyloxy oder Benzylthio bedeutet, daß der aromatische Ring unsubstituiert ist oder ein- bis dreifach halogeniert ist, wie 2-Cl, 2-F, 3-Cl, 4-Cl, 4-Br, 2,3-$Cl_2$, 2,4-$Cl_2$, 2,5-$Cl_2$, 2,6-$Cl_2$, 3,5-$Cl_2$, 2,4,6-$Cl_3$, 3,4,5-$Cl_3$ oder ein bis zwei Substituenten wie Nitro oder Methyl, 2-$NO_2$, 4-$NO_2$, 2-$CH_3$, 4-$CH_3$, 2,6-$(CH_3)_2$, 3,5-$(CH_3)_2$ trägt.

Für die Salze kommen beispielsweise folgende Kationen in Betracht: $Na^+$, $Li^+$, $K^+$, $Mg^{2+}$, $Fe^{3+}$, $NH_4^+$, einfach oder mehrfach alkylierte, hydroxyalkylierte und/oder arylierte Ammoniumkationen, wie Diisopropylammonium, Tetramethylammonium, Tetrabutylammonium, Trimethylbenzylammonium, N,N-Dimethylanilinium, Trimethyl-(2-hydroxyethyl)ammonium, wobei das Alkyl 1 bis 4 C-Atome hat und der Arylrest ein Phenylrest oder ein Benzylrest ist.

m bedeutet 1 bis 4, d.h. 1, 2, 3 oder 4.

Die Verbindungen der Formel I, gemäß Anspruch 1, in der $R^4$ Wasserstoff ist, werden z.B. dadurch hergestellt, daß man ein Benzisothiazol der Formel II

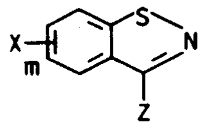

II,

in der X und m die in Anspruch 1 angegebene Bedeutung haben, mit Verbindungen der Formel III

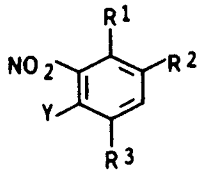

III,

in der $R^1$, $R^2$, $R^3$ die in Anspruch 1 angegebene Bedeutung haben, in Substanz oder in einem Lösungs- oder Verdünnungsmittel, gegebenenfalls in Anwesenheit einer organischen oder anorganischen Base bei Temperaturen zwischen -20 und 200 °C umsetzt, wobei Y und Z für $NH_2$ oder Halogen stehen und Y und Z voneinander verschieden sind.

In beiden Fällen erfolgt die Umsetzung unter Abspaltung von Halogenwasserstoff. In beiden Fällen ist deshalb die Verwendung von säurebindenden Mitteln bzw. Basen von Vorteil.

Als Basen bzw. säurebindende Mittel können organische oder anorganische Verbindungen verwendet werden, wie z.B. Alkali- und Erdalkalihydroxide (LiOH, NaOH, KOH, $Ca(OH)_2$), -oxide ($Na_2O$ $Li_2O$, CaO, MgO), -hydride (LiH, NaH, KH, $CaH_2$), -amide ($LiNH_2$, $NaNH_2$, $KNH_2$), -carbonate ($Li_2CO_3$, $Na_2CO_3$, $CaCO_3$), -hydrogencarbonate ($NaHCO_3$), -alkyle (ButylLi, $CH_3Li$, $CH_3MgCl$), -$C_1$-$C_5$-alkoholate ($NaOCH_3$, $NaOC_2H_5$, $KOC_2H_5$, KO-tert.-Butyl, $Mg(OCH_3)_2$), Amine, insbesondere tertiäre Amine (z.B. Trimethylamin, Triethylamin, Diisopropylethylamin, N-Methylpiperidin), Pyridin, substituierte Pyridine (Collidin, Lutidine, 4-Dimethylaminopyridin), bicyclische Amine.

Die Reaktionen können in Anwesenheit von reaktionsinerten Lösungs-oder Verdünnungsmitteln durchgeführt werden.

In Frage kommen beispielsweise aliphatische und aromatische Kohlenwasserstoffe wie Toluol, Xylole, Cyclohexan, Petrolether; halogenierte Kohlenwasserstoffe wie Chlorbenzol, Methylenchlorid, Chloroform; Ether und etherartige Verbindungen wie Dialkylether (Diethylether, Diisopropylether, tert.-Butylmethylether, usw.), Anisol, Dioxan, Tetrahydrofuran; Nitrile wie Acetonitril, Propionitril, N,N-dialkylierte Amide wie Dimethylformamid; Dimethylsulfoxid; Ketone wie Aceton, Diethylketon, tert.-Butylmethylketon, Methylethylketon, Alkohole wie Methanol, Ethanol, n- und iso-Propanol, Butanole, insbesondere tert.-Butanol und Gemische solcher Lösungsmittel untereinander.

Die Reaktionstemperatur läßt sich in weiten Grenzen ändern. Je nach Art der Substituenten X, $R^1$, $R^2$, $R^3$, $R^5$, $R^6$, $R^7$ und ihrer Zahl m arbeitet man vorteilhaft bei Temperaturen zwischen -20 °C und 200 °C bzw. beim Siedepunkt des Lösungsmittels bzw. Lösungsmittelgemisches.

In manchen Fällen kann es auch von Vorteil sein, die Reaktion unter Schutzgasatmosphäre und/oder wasserfreien Lösungsmitteln durchzuführen. Als Schutzgas eignen sich inerte Gase wie Helium, Argon, bevorzugt Stickstoff.

Die Ausgangsverbindungen sind bekannt oder nach bekannten Methoden einfach herstellbar. Die eingesetzten Halogenaromaten (bzw. einfach in diese umwandelbare Phenole) und Aniline sind bekannt.

Für 3-Amino- bzw. 3-Halogen-benzisothiazole seien folgende Literaturstellen genannt: DE-A 3112164, DE 1915387, DE 2609864.

Die Verbindungen der Formel I, gemäß Anspruch 1 mit der Maßgabe, daß $R^4$ verschieden von Wasserstoff ist, werden z.B. dadurch hergestellt, daß man ein 3-Anilino-benzisothiazol der Formel I, in der $R^4$ Wasserstoff ist und X, m, $R^1$, $R^2$, $R^3$, $R^5$, $R^6$, $R^7$ die in Anspruch 1 angegebene Bedeutung haben, mit einem Acylierungs- oder Sulfonierungsmittel der Formel IV,

$LR^4$ IV

in der

$R^4$ die in Anspruch 1 angegebene Bedeutung außer Wasserstoff hat und L für eine nucleophil verdrängbare Abgangsgruppe steht, in Substanz oder in Gegenwart eines Lösungs- oder Verdünnungsmittels und gegebenenfalls in Gegenwart einer organischen oder anorganischen Base bei Temperaturen zwischen -20 ° C und 150 ° C umsetzt.

Als nucleophil verdrängbare Gruppen eignen sich bevorzugt Halogenatome wie F, Cl, Br, J, Sulfonate (Methansulfonat, Benzolsulfonat u.a.), Alkoholate (Ethylat, Benzylat u. a.) und Carboxylate (Acetat u. a.).

Als Basen können die gleichen Verbindungen wie bei der Herstellung von Verbindungen der Formel I ($R^4$ = H) aus Verbindungen der Formeln II und III verwendet werden.

Die Reaktionstemperaturen liegen i. a. zwischen -20 ° C und 150 ° C bzw. am Siedepunkt des Lösungsmittels oder Lösungsmittelgemisches.

Bei der Reaktion können ein oder mehrere reaktionsinerte Lösungs- oder Verdünnungsmittel anwesend sein. In Frage kommen beispielsweise aliphatische und aromatische Kohlenwasserstoffe wie Toluol, Xylole, Cyclohexan, Petrolether; halogenierte Kohlenwasserstoffe wie Chlorbenzol, Methylenchlorid, Ethylenchlorid, Chloroform, Tetrachlorethylen; Ether und etherartige Verbindungen wie Dialkylether (Diethylether, Diisopropylether, tert.-Butylmethylether usw.) Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon, tert.-Butylmethylketon und Gemische solcher Lösungsmittel untereinander.

Zur Isolierung der neuen Verbindungen wird nach üblichen Methoden vorgegangen. Die anfallenden Produkte können durch Umkristallisieren, Extrahieren oder Chromatographieren gereinigt werden. Folgende Beispiele sollen die Herstellung illustrieren:

Beispiel 1

Zu einer Suspension von 19,5 g (0,1 Mol) 3-Amino-5-nitro-benzisothiazol und 32 g (0,105 Mol) 2,4-Dichlor-3,5-dinitro-benzotrifluorid in 300 ml Tetrahydrofuran/tert.-Butanol 1:1 wird bei 0 ° C bis 5 ° C eine Lösung von 23,5 g (0,21 Mol) Kalium-tert.-butylat in 250 ml tert.-Butanol innerhalb 1 h unter heftigem Rühren zugetropft. Nach einer weiteren Stunde bei 0 ° C wird auf Raumtemperatur erwärmt, mit Eisessig angesäuert, 500 ml Wasser zugegeben, der Niederschlag abgesaugt und mit kaltem Methanol nachgewaschen; 36,1 g (78 %) der Verbindung 1.46, gelbe Kristalle, Schmp. 168-170 ° C.

Beispiel 2

Zu einer Lösung von 3,4 g (0,01 Mol) 3-(2-Nitro-4-trifluormethylanilino)-benzisothiazol (Verbindung 1.20) in 25 ml Tetrahydrofuran werden 0,24 g (0,01 Mol) Natriumhydrid portionsweise zugegeben. Nach 15 Minuten werden bei Raumtemperatur 2,0 g (0,02 Mol) Essigsäureanhydrid zugetropft. Nach 48 h bei 20 ° C werden 150 ml Wasser zugegeben und es wird 3 mal mit je 100 ml Ether extrahiert. Die vereinigten Etherextrakte werden mit gesättigter NaCl-Lösung gewaschen, über $MgSO_4$ getrocknet und eingeengt. Der Rückstand wird chromatographiert (Kieselgel, Cyclohexan/Essigsäureethylester 19:1). Man erhält 1,3 g (34 %) der Verbindung 2.4, schwach gelbe Kristalle, Schmp. 108 bis 111 ° C, neben 1,4 g Ausgangsmaterial.

Beispiel 3

Zu einer noch heißen Lösung von 4,6 g (0,01 Mol) Verbindung 1.46 (5-Nitro-3-(2,6-dinitro-3-chlor-4-trifluormethylanilino)-benzisothiazol) in 150 ml Ethanol werden 6,5 ml einer 40 %igen wäßrigen Tetrabutylammoniumhydroxid-Lösung (0,01 Mol) zugetropft. Nach Abkühlen wird der Niederschlag abgesaugt, mit Ethanol und Diisopropylether nachgewaschen; 5,4 g (75 %) Verbindung 3.7, tiefdunkelrote Kristalle, Schmp. 143 ° C.

Tabelle 1

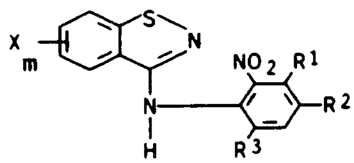

I,

| Nr. | $X_m$ | $R^1$ | $R^2$ | $R^3$ | Schmp. (°C) |
|---|---|---|---|---|---|
| 1.1 | - | Cl | $CF_3$ | $NO_2$ | 125 |
| 1.2 | - | Cl | CN | $NO_2$ | |
| 1.3 | - | Cl | $COOC_2H_5$ | $NO_2$ | |
| 1.4 | - | Cl | $CONH_2$ | $NO_2$ | |
| 1.5 | - | F | $CF_3$ | $NO_2$ | |
| 1.6 | - | $SCH_3$ | $CF_3$ | $NO_2$ | |
| 1.7 | - | $OCH_3$ | $CF_3$ | $NO_2$ | |
| 1.8 | - | $SC_2H_5$ | $CF_3$ | $NO_2$ | |
| 1.9 | - | H | $CF_3$ | $NO_2$ | 146-148 |
| 1.10 | - | H | CN | $NO_2$ | |
| 1.11 | - | H | Cl | $NO_2$ | |
| 1.12 | - | Cl | Cl | $NO_2$ | |
| 1.13 | - | H | $SO_2CH_3$ | $NO_2$ | |
| 1.14 | - | H | $COOCH_3$ | $NO_2$ | |
| 1.15 | - | H | $CH_3$ | $NO_2$ | |
| 1.16 | - | H | $NO_2$ | $CF_3$ | 152-158 |
| 1.17 | - | H | $NO_2$ | Cl | |
| 1.18 | - | H | $NO_2$ | CHMeEt | |
| 1.19 | - | Cl | Cl | Cl | |
| 1.20 | - | H | $CF_3$ | H | 130-133 |
| 1.21 | - | $CF_3$ | $CF_3$ | $NO_2$ | |
| 1.22 | 4-$NO_2$ | Cl | $CF_3$ | $NO_2$ | |
| 1.23 | 4-$NO_2$ | F | $CF_3$ | $NO_2$ | |
| 1.24 | 4-$NO_2$ | H | $CF_3$ | $NO_2$ | |
| 1.25 | 4-$NO_2$ | H | $NO_2$ | $CF_3$ | |
| 1.26 | 4-$NO_2$ | H | $NO_2$ | Cl | |
| 1.27 | 4-$NO_2$ | Cl | Cl | $NO_2$ | |
| 1.28 | 4-$NO_2$ | Cl | CN | $NO_2$ | |
| 1.29 | 4-$NO_2$ | Cl | $COOC_2H_5$ | $NO_2$ | |
| 1.30 | 4-Cl | Cl | $CF_3$ | $NO_2$ | 173-178 |
| 1.31 | 4-Cl | F | $CF_3$ | $NO_2$ | |
| 1.32 | 4-Cl | H | $CF_3$ | $NO_2$ | |
| 1.33 | 4-Cl | H | $NO_2$ | $CF_3$ | 147-149 |
| 1.34 | 4-Cl | H | $NO_2$ | Cl | |

Tabelle 1 (Fortsetzung)

| Nr. | $X_m$ | $R^1$ | $R^2$ | $R^3$ | Schmp. (°C) |
|---|---|---|---|---|---|
| 1.35 | 4-Cl | Cl | Cl | $NO_2$ | |
| 1.36 | 4-Cl | Cl | CN | $NO_2$ | |
| 1.37 | 4-Cl | Cl | $COOC_2H_5$ | $NO_2$ | |
| 1.38 | $4-CF_3$ | Cl | $CF_3$ | $NO_2$ | |
| 1.39 | $4-CF_3$ | F | $CF_3$ | $NO_2$ | |
| 1.40 | $4-CF_3$ | H | $CF_3$ | $NO_2$ | |
| 1.41 | $4-CF_3$ | H | $NO_2$ | $CF_3$ | |
| 1.42 | $4-CF_3$ | H | $NO_2$ | Cl | |
| 1.43 | $4-CF_3$ | Cl | Cl | $NO_2$ | |
| 1.44 | $4-CF_3$ | Cl | CN | $NO_2$ | |
| 1.45 | $4-CF_3$ | Cl | $COOC_2H_5$ | $NO_2$ | |
| 1.46 | $5-NO_2$ | Cl | $CF_3$ | $NO_2$ | 168-170 |
| 1.47 | $5-NO_2$ | F | $CF_3$ | $NO_2$ | 76- 84 |
| 1.48 | $5-NO_2$ | Br | $CF_3$ | $NO_2$ | |
| 1.49 | $5-NO_2$ | $CF_3$ | $CF_3$ | $NO_2$ | |
| 1.50 | $5-NO_2$ | $SCH_3$ | $CF_3$ | $NO_2$ | |
| 1.51 | $5-NO_2$ | $OCH_3$ | $CF_3$ | $NO_2$ | |
| 1.52 | $5-NO_2$ | $SC_2H_5$ | $CF_3$ | $NO_2$ | |
| 1.53 | $5-NO_2$ | $OC_2H_5$ | $CF_3$ | $NO_2$ | |
| 1.54 | $5-NO_2$ | $OC_4H_9$-n | $CF_3$ | $NO_2$ | |
| 1.55 | $5-NO_2$ | $OCF_3$ | $CF_3$ | $NO_2$ | |
| 1.56 | $5-NO_2$ | $OCF_2CHF_2$ | $CF_3$ | $NO_2$ | |
| 1.57 | $5-NO_2$ | $OCH_2C_6H_5$ | $CF_3$ | $NO_2$ | |
| 1.58 | $5-NO_2$ | $OC_6H_5$ | $CF_3$ | $NO_2$ | |
| 1.59 | $5-NO_2$ | $SCH_2C_6H_5$ | $CF_3$ | $NO_2$ | |
| 1.60 | $5-NO_2$ | $SC_6H_5$ | $CF_3$ | $NO_2$ | |
| 1.61 | $5-NO_2$ | $OC_6H_4$-p-Cl | $CF_3$ | $NO_2$ | |
| 1.62 | $5-NO_2$ | $SC_6H_4$-p-Cl | $CF_3$ | $NO_2$ | |
| 1.63 | $5-NO_2$ | H | $CF_3$ | $NO_2$ | 174-178 |
| 1.64 | $5-NO_2$ | H | H | F | |
| 1.65 | $5-NO_2$ | H | Br | $NO_2$ | |
| 1.66 | $5-NO_2$ | H | Cl | $NO_2$ | |
| 1.67 | $5-NO_2$ | H | CN | $NO_2$ | >220 |
| 1.68 | $5-NO_2$ | H | $CONH_2$ | $NO_2$ | |
| 1.69 | $5-NO_2$ | H | $CON(CH_3)_2$ | $NO_2$ | |
| 1.70 | $5-NO_2$ | H | COOH | $NO_2$ | |
| 1.71 | $5-NO_2$ | H | $COOCH_3$ | $NO_2$ | 145-155 |
| 1.72 | $5-NO_2$ | Cl | CN | $NO_2$ | |
| 1.73 | $5-NO_2$ | Cl | $COOC_2H_5$ | $NO_2$ | 179-185 |

Tabelle 1 (Fortsetzung)

| Nr. | $X_m$ | $R^1$ | $R^2$ | $R^3$ | Schmp. (°C) |
|---|---|---|---|---|---|
| 1.74 | 5-$NO_2$ | Cl | $CONH_2$ | $NO_2$ | |
| 1.75 | 5-$NO_2$ | H | $SO_2CH_3$ | $NO_2$ | >220 |
| 1.76 | 5-$NO_2$ | H | $SO_2NH_2$ | $NO_2$ | |
| 1.77 | 5-$NO_2$ | H | $SO_2N(CH_3)_2$ | $NO_2$ | |
| 1.78 | 5-$NO_2$ | H | $CH_3$ | $NO_2$ | >220 |
| 1.79 | 5-$NO_2$ | H | $C(CH_3)_3$ | $NO_2$ | >220 |
| 1.80 | 5-$NO_2$ | H | $OCF_3$ | $NO_2$ | |
| 1.81 | 5-$NO_2$ | H | $OCF_2CHF_2$ | $NO_2$ | |
| 1.82 | 5-$NO_2$ | H | $NO_2$ | $CF_3$ | 192-194 |
| 1.83 | 5-$NO_2$ | H | $NO_2$ | Cl | 178-180 |
| 1.84 | 5-$NO_2$ | H | $NO_2$ | $CH_3$ | |
| 1.85 | 5-$NO_2$ | H | $NO_2$ | CHMeEt | 84-90 |
| 1.86 | 5-$NO_2$ | Cl | Cl | $NO_2$ | >220 |
| 1.87 | 5-$NO_2$ | Cl | Cl | Cl | 204-212 |
| 1.88 | 5-$NO_2$ | H | $CF_3$ | H | >220 |
| 1.89 | 5-$CF_3$ | Cl | $CF_3$ | $NO_2$ | 134-137 |
| 1.90 | 5-$CF_3$ | Cl | CN | $NO_2$ | |
| 1.91 | 5-$CF_3$ | Cl | $COOC_2H_5$ | $NO_2$ | |
| 1.92 | 5-$CF_3$ | Cl | $CONH_2$ | $NO_2$ | |
| 1.93 | 5-$CF_3$ | F | $CF_3$ | $NO_2$ | |
| 1.94 | 5-$CF_3$ | $OCH_3$ | $CF_3$ | $NO_2$ | |
| 1.95 | 5-$CF_3$ | H | $CF_3$ | $NO_2$ | 125-129 |
| 1.96 | 5-$CF_3$ | H | CN | $NO_2$ | |
| 1.97 | 5-$CF_3$ | H | Cl | $NO_2$ | |
| 1.98 | 5-$CF_3$ | Cl | Cl | $NO_2$ | |
| 1.99 | 5-$CF_3$ | H | $SO_2CH_3$ | $NO_2$ | |
| 1.100 | 5-$CF_3$ | H | $COOCH_3$ | $NO_2$ | |
| 1.101 | 5-$CF_3$ | H | $CH_3$ | $NO_2$ | |
| 1.102 | 5-$CF_3$ | H | $NO_2$ | $CF_3$ | 118-119 |
| 1.103 | 5-$CF_3$ | H | $NO_2$ | Cl | |
| 1.104 | 5-$CF_3$ | H | $NO_2$ | CHMeEt | |
| 1.105 | 5-$CF_3$ | Cl | Cl | Cl | |
| 1.106 | 5-Cl | Cl | $CF_3$ | $NO_2$ | |
| 1.107 | 5-Cl | Cl | CN | $NO_2$ | |
| 1.108 | 5-Cl | Cl | $COOC_2H_5$ | $NO_2$ | |
| 1.109 | 5-Cl | Cl | $CONH_2$ | $NO_2$ | |
| 1.110 | 5-Cl | F | $CF_3$ | $NO_2$ | |
| 1.111 | 5-Cl | $OCH_3$ | $CF_3$ | $NO_2$ | |
| 1.112 | 5-Cl | H | $CF_3$ | $NO_2$ | |

Tabelle 1 (Fortsetzung)

| Nr. | $X_m$ | $R^1$ | $R^2$ | $R^3$ | Schmp. (°C) |
|---|---|---|---|---|---|
| 1.113 | 5-Cl | H | CN | $NO_2$ | |
| 1.114 | 5-Cl | H | Cl | $NO_2$ | |
| 1.115 | 5-Cl | Cl | Cl | $NO_2$ | |
| 1.116 | 5-Cl | H | $SO_2CH_3$ | $NO_2$ | |
| 1.117 | 5-Cl | H | $COOCH_3$ | $NO_2$ | |
| 1.118 | 5-Cl | H | $CH_3$ | $NO_2$ | |
| 1.119 | 5-Cl | H | $NO_2$ | $CF_3$ | |
| 1.120 | 5-Cl | H | $NO_2$ | Cl | |
| 1.121 | 5-Cl | H | $NO_2$ | CHMeEt | |
| 1.122 | 5-Cl | Cl | Cl | Cl | |
| 1.123 | 6-$NO_2$ | Cl | $CF_3$ | $NO_2$ | |
| 1.124 | 6-$NO_2$ | Cl | CN | $NO_2$ | |
| 1.125 | 6-$NO_2$ | Cl | $COOC_2H_5$ | $NO_2$ | |
| 1.126 | 6-$NO_2$ | Cl | $CONH_2$ | $NO_2$ | |
| 1.127 | 6-$NO_2$ | F | $CF_3$ | $NO_2$ | |
| 1.128 | 6-$NO_2$ | $OCH_3$ | $CF_3$ | $NO_2$ | |
| 1.129 | 6-$NO_2$ | H | $CF_3$ | $NO_2$ | |
| 1.130 | 6-$NO_2$ | H | CN | $NO_2$ | |
| 1.131 | 6-$NO_2$ | H | Cl | $NO_2$ | |
| 1.132 | 6-$NO_2$ | Cl | Cl | $NO_2$ | |
| 1.133 | 6-$NO_2$ | H | $SO_2CH_3$ | $NO_2$ | |
| 1.134 | 6-$NO_2$ | H | $COOCH_3$ | $NO_2$ | |
| 1.135 | 6-$NO_2$ | H | $CH_3$ | $NO_2$ | |
| 1.136 | 6-$NO_2$ | H | $NO_2$ | $CF_3$ | |
| 1.137 | 6-$NO_2$ | H | $NO_2$ | Cl | |
| 1.138 | 6-$NO_2$ | H | $NO_2$ | CHMeEt | |
| 1.139 | 6-$NO_2$ | Cl | Cl | Cl | |
| 1.140 | 6-$CF_3$ | Cl | $CF_3$ | $NO_2$ | |
| 1.141 | 6-$CF_3$ | Cl | CN | $NO_2$ | |
| 1.142 | 6-$CF_3$ | Cl | $COOC_2H_5$ | $NO_2$ | |
| 1.143 | 6-$CF_3$ | Cl | $CONH_2$ | $NO_2$ | |
| 1.144 | 6-$CF_3$ | F | $CF_3$ | $NO_2$ | |
| 1.145 | 6-$CF_3$ | $OCH_3$ | $CF_3$ | $NO_2$ | |
| 1.146 | 6-$CF_3$ | H | $CF_3$ | $NO_2$ | |
| 1.147 | 6-$CF_3$ | H | CN | $NO_2$ | |
| 1.148 | 6-$CF_3$ | H | Cl | $NO_2$ | |
| 1.149 | 6-$CF_3$ | Cl | Cl | $NO_2$ | |
| 1.150 | 6-$CF_3$ | H | $SO_2CH_3$ | $NO_2$ | |
| 1.151 | 6-$CF_3$ | H | $COOCH_3$ | $NO_2$ | |

Tabelle 1 (Fortsetzung)

| Nr. | $X_m$ | $R^1$ | $R^2$ | $R^3$ | Schmp. (°C) |
|---|---|---|---|---|---|
| 1.152 | 6-$CF_3$ | H | $CH_3$ | $NO_2$ | |
| 1.153 | 6-$CF_3$ | H | $NO_2$ | $CF_3$ | |
| 1.154 | 6-$CF_3$ | H | $NO_2$ | Cl | |
| 1.155 | 6-$CF_3$ | H | $NO_2$ | CHMeEt | |
| 1.156 | 6-$CF_3$ | Cl | Cl | Cl | |
| 1.157 | 6-Cl | Cl | $CF_3$ | $NO_2$ | 169-172 |
| 1.158 | 6-Cl | Cl | CN | $NO_2$ | |
| 1.159 | 6-Cl | Cl | $COOC_2H_5$ | $NO_2$ | |
| 1.160 | 6-Cl | Cl | $CONH_2$ | $NO_2$ | |
| 1.161 | 6-Cl | F | $CF_3$ | $NO_2$ | |
| 1.162 | 6-Cl | $OCH_3$ | $CF_3$ | $NO_2$ | |
| 1.163 | 6-Cl | H | $CF_3$ | $NO_2$ | |
| 1.164 | 6-Cl | H | CN | $NO_2$ | |
| 1.165 | 6-Cl | H | Cl | $NO_2$ | |
| 1.166 | 6-Cl | Cl | Cl | $NO_2$ | |
| 1.167 | 6-Cl | H | $SO_2CH_3$ | $NO_2$ | |
| 1.168 | 6-Cl | H | $COOCH_3$ | $NO_2$ | |
| 1.169 | 6-Cl | H | $CH_3$ | $NO_2$ | |
| 1.170 | 6-Cl | H | $NO_2$ | $CF_3$ | 146-147 |
| 1.171 | 6-Cl | H | $NO_2$ | Cl | |
| 1.172 | 6-Cl | H | $NO_2$ | CHMeEt | |
| 1.173 | 6-Cl | Cl | Cl | Cl | |
| 1.174 | 7-$NO_2$ | Cl | $CF_3$ | $NO_2$ | |
| 1.175 | 7-$NO_2$ | F | $CF_3$ | $NO_2$ | |
| 1.176 | 7-$NO_2$ | H | $CF_3$ | $NO_2$ | |
| 1.177 | 7-$NO_2$ | H | $NO_2$ | $CF_3$ | |
| 1.178 | 7-$NO_2$ | H | $NO_2$ | Cl | |
| 1.179 | 7-$NO_2$ | Cl | Cl | $NO_2$ | |
| 1.180 | 7-$NO_2$ | Cl | CN | $NO_2$ | |
| 1.181 | 7-$NO_2$ | Cl | $COOC_2H_5$ | $NO_2$ | |
| 1.182 | 7-Cl | Cl | $CF_3$ | $NO_2$ | |
| 1.183 | 7-Cl | F | $CF_3$ | $NO_2$ | |
| 1.184 | 7-Cl | H | $CF_3$ | $NO_2$ | |
| 1.185 | 7-Cl | H | $NO_2$ | $CF_3$ | |
| 1.186 | 7-Cl | H | $NO_2$ | Cl | |
| 1.187 | 7-Cl | Cl | Cl | $NO_2$ | |
| 1.188 | 7-Cl | Cl | CN | $NO_2$ | |
| 1.189 | 7-Cl | Cl | $COOC_2H_5$ | $NO_2$ | |
| 1.190 | 7-$CF_3$ | Cl | $CF_3$ | $NO_2$ | |

Tabelle 1 (Fortsetzung)

| Nr. | $X_m$ | $R^1$ | $R^2$ | $R^3$ | Schmp. (°C) |
|---|---|---|---|---|---|
| 1.191 | 7-$CF_3$ | F | $CF_3$ | $NO_2$ | |
| 1.192 | 7-$CF_3$ | H | $CF_3$ | $NO_2$ | |
| 1.193 | 7-$CF_3$ | H | $NO_2$ | $CF_3$ | |
| 1.194 | 7-$CF_3$ | H | $NO_2$ | Cl | |
| 1.195 | 7-$CF_3$ | Cl | Cl | $NO_2$ | |
| 1.196 | 7-$CF_3$ | Cl | CN | $NO_2$ | |
| 1.197 | 7-$CF_3$ | Cl | $COOC_2H_5$ | $NO_2$ | |
| 1.198 | 4-$OCH_3$ | Cl | $CF_3$ | $NO_2$ | |
| 1.199 | 4-$OCH_3$ | H | $NO_2$ | $CF_3$ | |
| 1.200 | 5-$OCH_3$ | Cl | $CF_3$ | $NO_2$ | |
| 1.201 | 5-$OCH_3$ | H | $NO_2$ | $CF_3$ | |
| 1.202 | 6-$OCH_3$ | Cl | $CF_3$ | $NO_2$ | |
| 1.203 | 6-$OCH_3$ | H | $NO_2$ | $CF_3$ | |
| 1.204 | 7-$OCH_3$ | Cl | $CF_3$ | $NO_2$ | |
| 1.205 | 7-$OCH_3$ | H | $NO_2$ | $CF_3$ | |
| 1.206 | 4-Br | Cl | $CF_3$ | $NO_2$ | |
| 1.207 | 4-Br | H | $NO_2$ | $CF_3$ | |
| 1.208 | 5-Br | Cl | $CF_3$ | $NO_2$ | |
| 1.209 | 5-Br | H | $NO_2$ | $CF_3$ | |
| 1.210 | 6-Br | Cl | $CF_3$ | $NO_2$ | |
| 1.211 | 6-Br | H | $NO_2$ | $CF_3$ | |
| 1.212 | 7-Br | Cl | $CF_3$ | $NO_2$ | |
| 1.213 | 7-Br | H | $NO_2$ | $CF_3$ | |
| 1.214 | 4-F | Cl | $CF_3$ | $NO_2$ | |
| 1.215 | 4-F | H | $NO_2$ | $CF_3$ | |
| 1.216 | 5-F | Cl | $CF_3$ | $NO_2$ | |
| 1.217 | 5-F | H | $NO_2$ | $CF_3$ | |
| 1.218 | 6-F | Cl | $CF_3$ | $NO_2$ | |
| 1.219 | 6-F | H | $NO_2$ | $CF_3$ | |
| 1.220 | 7-F | Cl | $CF_3$ | $NO_2$ | |
| 1.221 | 7-F | H | $NO_2$ | $CF_3$ | |
| 1.222 | 4-cyclo-$C_3H_5$ | Cl | $CF_3$ | $NO_2$ | |
| 1.223 | 4-cyclo-$C_3H_5$ | H | $NO_2$ | $CF_3$ | |
| 1.224 | 5-cyclo-$C_3H_5$ | Cl | $CF_3$ | $NO_2$ | |
| 1.225 | 5-cyclo-$C_3H_5$ | H | $NO_2$ | $CF_3$ | |
| 1.226 | 6-cyclo-$C_3H_5$ | Cl | $CF_3$ | $NO_2$ | |
| 1.227 | 6-cyclo-$C_3H_5$ | H | $NO_2$ | $CF_3$ | |
| 1.228 | 7-cyclo-$C_3H_5$ | Cl | $CF_3$ | $NO_2$ | |
| 1.229 | 7-cyclo-$C_3H_5$ | H | $NO_2$ | $CF_3$ | |

Tabelle 1 (Fortsetzung)

| Nr. | $X_m$ | $R^1$ | $R^2$ | $R^3$ | Schmp. (°C) |
|---|---|---|---|---|---|
| 1.230 | 5-$OCF_2CHF_2$ | Cl | $CF_3$ | $NO_2$ | |
| 1.231 | 5-$OCF_2CHF_2$ | H | $NO_2$ | $CF_3$ | |
| 1.232 | 6-$OCF_2CHF_2$ | Cl | $CF_3$ | $NO_2$ | |
| 1.233 | 6-$OCF_2CHF_2$ | H | $NO_2$ | $CF_3$ | |
| 1.234 | 5-OPh | Cl | $CF_3$ | $NO_2$ | |
| 1.235 | 5-OPh | H | $NO_2$ | $CF_3$ | |
| 1.236 | 5-SPh | Cl | $CF_3$ | $NO_2$ | |
| 1.237 | 5-SPh | H | $NO_2$ | $CF_3$ | |
| 1.238 | 4-$CH_3$ | Cl | $CF_3$ | $NO_2$ | |
| 1.239 | 4-$CH_3$ | H | $NO_2$ | $CF_3$ | |
| 1.240 | 5-$CH_3$ | Cl | $CF_3$ | $NO_2$ | |
| 1.241 | 5-$CH_3$ | H | $NO_2$ | $CF_3$ | |
| 1.242 | 6-$CH_3$ | Cl | $CF_3$ | $NO_2$ | |
| 1.243 | 6-$CH_3$ | H | $NO_2$ | $CF_3$ | |
| 1.244 | 7-$CH_3$ | Cl | $CF_3$ | $NO_2$ | |
| 1.245 | 7-$CH_3$ | H | $NO_2$ | $CF_3$ | |
| 1.246 | 5-$OCF_3$ | Cl | $CF_3$ | $NO_2$ | |
| 1.247 | 5-$OCF_3$ | H | $NO_2$ | $CF_3$ | |
| 1.248 | 6-$OCF_3$ | Cl | $CF_3$ | $NO_2$ | |
| 1.249 | 6-$OCF_3$ | H | $NO_2$ | $CF_3$ | |
| 1.250 | 4-CN | Cl | $CF_3$ | $NO_2$ | |
| 1.251 | 4-CN | H | $NO_2$ | $CF_3$ | |
| 1.252 | 5-CN | Cl | $CF_3$ | $NO_2$ | |
| 1.253 | 5-CN | H | $NO_2$ | $CF_3$ | |
| 1.254 | 6-CN | Cl | $CF_3$ | $NO_2$ | |
| 1.255 | 6-CN | H | $NO_2$ | $CF_3$ | |
| 1.256 | 7-CN | Cl | $CF_3$ | $NO_2$ | |
| 1.257 | 7-CN | H | $NO_2$ | $CF_3$ | |
| 1.258 | 6-$C(CH_3)_3$ | Cl | $CF_3$ | $NO_2$ | |
| 1.259 | 6-$C(CH_3)_3$ | H | $NO_2$ | $CF_3$ | |
| 1.260 | 4-$SO_2CH_3$ | Cl | $CF_3$ | $NO_2$ | |
| 1.261 | 4-$SO_2CH_3$ | H | $NO_2$ | $CF_3$ | |
| 1.262 | 5-$SO_2CH_3$ | Cl | $CF_3$ | $NO_2$ | |
| 1.263 | 5-$SO_2CH_3$ | H | $NO_2$ | $CF_3$ | |
| 1.264 | 6-$SO_2CH_3$ | Cl | $CF_3$ | $NO_2$ | |
| 1.265 | 6-$SO_2CH_3$ | H | $NO_2$ | $CF_3$ | |
| 1.266 | 7-$SO_2CH_3$ | Cl | $CF_3$ | $NO_2$ | |
| 1.267 | 7-$SO_2CH_3$ | H | $NO_2$ | $CF_3$ | |
| 1.268 | 6-$SCH_3$ | Cl | $CF_3$ | $NO_2$ | |

Tabelle 1 (Fortsetzung)

| Nr. | $X_m$ | $R^1$ | $R^2$ | $R^3$ | Schmp. (°C) |
|---|---|---|---|---|---|
| 1.269 | 6-$SCH_3$ | H | $NO_2$ | $CF_3$ | |
| 1.270 | 4,5-$Cl_2$ | Cl | $CF_3$ | $NO_2$ | |
| 1.271 | 4,5-$Cl_2$ | F | $CF_3$ | $NO_2$ | |
| 1.272 | 4,5-$Cl_2$ | H | $CF_3$ | $NO_2$ | |
| 1.273 | 4,5-$Cl_2$ | H | $NO_2$ | $CF_3$ | |
| 1.274 | 4,5-$Cl_2$ | H | $NO_2$ | Cl | |
| 1.275 | 4,5-$Cl_2$ | | Cl | $NO_2$ | |
| 1.276 | 4,5-$Cl_2$ | Cl | CN | $NO_2$ | |
| 1.277 | 4,5-$Cl_2$ | Cl | $COOC_2H_5$ | $NO_2$ | |
| 1.278 | 4-Cl, 5-$CF_3$ | Cl | $CF_3$ | $NO_2$ | |
| 1.279 | 4-Cl, 5-$CF_3$ | F | $CF_3$ | $NO_2$ | |
| 1.280 | 4-Cl, 5-$CF_3$ | H | $CF_3$ | $NO_2$ | |
| 1.281 | 4-Cl, 5-$CF_3$ | H | $NO_2$ | $CF_3$ | |
| 1.282 | 4-Cl, 5-$CF_3$ | H | $NO_2$ | Cl | |
| 1.283 | 4-Cl, 5-$CF_3$ | Cl | Cl | $NO_2$ | |
| 1.284 | 4-Cl, 5-$CF_3$ | Cl | CN | $NO_2$ | |
| 1.285 | 4-Cl, 5-$CF_3$ | Cl | $COOC_2H_5$ | $NO_2$ | |
| 1.286 | 7-Cl, 5-$CF_3$ | Cl | $CF_3$ | $NO_2$ | |
| 1.287 | 7-Cl, 5-$CF_3$ | F | $CF_3$ | $NO_2$ | |
| 1.288 | 7-Cl, 5-$CF_3$ | H | $CF_3$ | $NO_2$ | |
| 1.289 | 7-Cl, 5-$CF_3$ | H | $NO_2$ | $CF_3$ | |
| 1.290 | 7-Cl, 5-$CF_3$ | H | $NO_2$ | Cl | |
| 1.291 | 7-Cl, 5-$CF_3$ | Cl | Cl | $NO_2$ | |
| 1.292 | 7-Cl, 5-$CF_3$ | Cl | CN | $NO_2$ | |
| 1.293 | 7-Cl, 5-$CF_3$ | Cl | $COOC_2H_5$ | $NO_2$ | |
| 1.294 | 5,6-$Cl_2$ | Cl | $CF_3$ | $NO_2$ | |
| 1.295 | 5,6-$Cl_2$ | F | $CF_3$ | $NO_2$ | |
| 1.296 | 5,6-$Cl_2$ | H | $CF_3$ | $NO_2$ | |
| 1.297 | 5,6-$Cl_2$ | H | $NO_2$ | $CF_3$ | |
| 1.298 | 5,6-$Cl_2$ | H | $NO_2$ | Cl | |
| 1.299 | 5,6-$Cl_2$ | Cl | Cl | $NO_2$ | |
| 1.300 | 5,6-$Cl_2$ | Cl | CN | $NO_2$ | |
| 1.301 | 5,6-$Cl_2$ | Cl | $COOC_2H_5$ | $NO_2$ | |
| 1.302 | 5,7-$(NO_2)_2$ | Cl | $CF_3$ | $NO_2$ | |
| 1.303 | 5,7-$(NO_2)_2$ | F | $CF_3$ | $NO_2$ | |
| 1.204 | 5,7-$(NO_2)_2$ | H | $CF_3$ | $NO_2$ | |
| 1.305 | 5,7-$(NO_2)_2$ | H | $NO_2$ | $CF_3$ | |
| 1.306 | 5,7-$(NO_2)_2$ | H | $NO_2$ | Cl | |
| 1.307 | 5,7-$(NO_2)_2$ | Cl | Cl | $NO_2$ | |

Tabelle 1 (Fortsetzung)

| Nr. | $X_m$ | $R^1$ | $R^2$ | $R^3$ | Schmp. (°C) |
|---|---|---|---|---|---|
| 1.308 | 5,7-$(NO_2)_2$ | Cl | CN | $NO_2$ | |
| 1.309 | 5,7-$(NO_2)_2$ | Cl | $COOC_2H_5$ | $NO_2$ | |
| 1.310 | 5-$NO_2$, 7-Cl | Cl | $CF_3$ | $NO_2$ | |
| 1.311 | 5-$NO_2$, 7-Cl | F | $CF_3$ | $NO_2$ | |
| 1.312 | 5-$NO_2$, 7-Cl | H | $CF_3$ | $NO_2$ | |
| 1.313 | 5-$NO_2$, 7-Cl | H | $NO_2$ | $CF_3$ | |
| 1.314 | 5-$NO_2$, 7-Cl | H | $NO_2$ | Cl | |
| 1.315 | 5-$NO_2$, 7-Cl | Cl | Cl | $NO_2$ | |
| 1.316 | 5-$NO_2$, 7-Cl | Cl | CN | $NO_2$ | |
| 1.317 | 5-$NO_2$, 7-Cl | Cl | $COOC_2H_5$ | $NO_2$ | |
| 1.318 | 4-Cl, 5-$NO_2$ | Cl | $CF_3$ | $NO_2$ | |
| 1.319 | 4-Cl, 5-$NO_2$ | F | $CF_3$ | $NO_2$ | |
| 1.320 | 4-Cl, 5-$NO_2$ | H | $CF_3$ | $NO_2$ | |
| 1.321 | 4-Cl, 5-$NO_2$ | H | $NO_2$ | $CF_3$ | |
| 1.322 | 4-Cl, 5-$NO_2$ | H | $NO_2$ | Cl | |
| 1.323 | 4-Cl, 5-$NO_2$ | Cl | Cl | $NO_2$ | |
| 1.324 | 4-Cl, 5-$NO_2$ | Cl | CN | $NO_2$ | |
| 1.325 | 4-Cl, 5-$NO_2$ | Cl | $COOC_2H_5$ | $NO_2$ | |
| 1.326 | 4-Cl, 7-$NO_2$ | Cl | $CF_3$ | $NO_2$ | |
| 1.327 | 4-Cl, 7-$NO_2$ | F | $CF_3$ | $NO_2$ | |
| 1.228 | 4-Cl, 7-$NO_2$ | H | $CF_3$ | $NO_2$ | |
| 1.329 | 4-Cl, 7-$NO_2$ | H | $NO_2$ | $CF_3$ | |
| 1.330 | 4-Cl, 7-$NO_2$ | H | $NO_2$ | Cl | |
| 1.331 | 4-Cl, 7-$NO_2$ | Cl | Cl | $NO_2$ | |
| 1.332 | 4-Cl, 7-$NO_2$ | Cl | CN | $NO_2$ | |
| 1.333 | 4-Cl, 7-$NO_2$ | Cl | $COOC_2H_5$ | $NO_2$ | |
| 1.334 | 6-Cl, 5,7-$(NO_2)_2$ | Cl | $CF_3$ | $NO_2$ | |
| 1.335 | 6-Cl, 5,7-$(NO_2)_2$ | F | $CF_3$ | $NO_2$ | |
| 1.336 | 6-Cl, 5,7-$(NO_2)_2$ | H | $CF_3$ | $NO_2$ | |
| 1.337 | 6-Cl, 5,7-$(NO_2)_2$ | H | $NO_2$ | $CF_3$ | |
| 1.338 | 6-Cl, 5,7-$(NO_2)_2$ | H | $NO_2$ | Cl | |
| 1.339 | 6-Cl, 5,7-$(NO_2)_2$ | Cl | Cl | $NO_2$ | |
| 1.340 | 6-Cl, 5,7-$(NO_2)_2$ | Cl | CN | $NO_2$ | |
| 1.341 | 6-Cl, 5,7-$(NO_2)_2$ | Cl | $COOC_2H_5$ | $NO_2$ | |
| 1.342 | 6-F, 5,7-$(NO_2)_2$ | Cl | $CF_3$ | $NO_2$ | |
| 1.343 | 6-F, 5,7-$(NO_2)_2$ | H | $NO_2$ | $CF_3$ | |
| 1.344 | 6-Br, 5,7-$(NO_2)_2$ | Cl | $CF_3$ | $NO_2$ | |
| 1.345 | 6-Br, 5,7-$(NO_2)_2$ | H | $NO_2$ | $CF_3$ | |
| 1.346 | 6-CN, 5,7-$(NO_2)_2$ | Cl | $CF_3$ | $NO_2$ | |

Tabelle 1 (Fortsetzung)

| Nr. | $X_m$ | $R^1$ | $R^2$ | $R^3$ | Schmp. (°C) |
|---|---|---|---|---|---|
| 1.347 | 6-CN, 5,7-$(NO_2)_2$ | H | $NO_2$ | $CF_3$ | |
| 1.348 | 6-$OCH_3$, 5,7-$(NO_2)_2$ | Cl | $CF_3$ | $NO_2$ | |
| 1.349 | 6-$OCH_3$, 5,7-$(NO_2)_2$ | Cl | $NO_2$ | $CF_3$ | |
| 1.350 | 6-$SC_2H_5$, 5,7-$(NO_2)_2$ | Cl | $CF_3$ | $NO_2$ | |
| 1.351 | 6-$SC_2H_5$, 5,7-$(NO_2)_2$ | H | $NO_2$ | $CF_3$ | |
| 1.352 | 6-OPh, 5,7-$(NO_2)_2$ | Cl | $CF_3$ | $NO_2$ | |
| 1.353 | 6-OPh, 5,7-$(NO_2)_2$ | H | $NO_2$ | $CF_3$ | |
| 1.354 | 6-SPh, 5,7-$(NO_2)_2$ | Cl | $CF_3$ | $NO_2$ | |
| 1.355 | 6-SPh, 5,7-$(NO_2)_2$ | H | $NO_2$ | $CF_3$ | |
| 1.356 | 4-$OCH_3$, 5-$NO_2$ | Cl | $CF_3$ | $NO_2$ | |
| 1.357 | 4-$OCH_3$, 5-$NO_2$ | H | $NO_2$ | $CF_3$ | |
| 1.358 | 4-CN, 5-$NO_2$ | Cl | $CF_3$ | $NO_2$ | |
| 1.359 | 4-CN, 5-$NO_2$ | H | $NO_2$ | $CF_3$ | |
| 1.360 | 4-$OCH_3$, 7-$NO_2$ | Cl | $CF_3$ | $NO_2$ | |
| 1.361 | 4-$OCH_3$, 7-$NO_2$ | H | $NO_2$ | $CF_3$ | |
| 1.362 | 4-CN, 7-$NO_2$ | Cl | $CF_3$ | $NO_2$ | |
| 1.363 | 4-CN, 7-$NO_2$ | H | $NO_2$ | $CF_3$ | |
| 1.364 | 4-$OCH_3$, 5-Cl | Cl | $CF_3$ | $NO_2$ | |
| 1.365 | 4-$OCH_3$, 5-Cl | H | $NO_2$ | $CF_3$ | |
| 1.366 | 5-$NO_2$, 6-Cl | Cl | $CF_3$ | $NO_2$ | |
| 1.367 | 5-$NO_2$, 6-Cl | H | $NO_2$ | $CF_3$ | |
| 1.368 | 5-$NO_2$, 6-$CF_3$ | Cl | $CF_3$ | $NO_3$ | |
| 1.369 | 5-$NO_2$, 6-$CF_3$ | H | $NO_2$ | $CF_3$ | |
| 1.370 | 5-$NO_2$, 4-$CF_3$ | Cl | $CF_3$ | $NO_2$ | |
| 1.371 | 5-$NO_2$, 4-$CF_3$ | H | $NO_2$ | $CF_3$ | |
| 1.372 | 5,7-$(CF_3)_2$ | Cl | $CF_3$ | $NO_2$ | |
| 1.373 | 5,7-$(CF_3)_2$ | H | $NO_2$ | $CF_3$ | |
| 1.374 | 5-Cl, 7-$CF_3$ | Cl | $CF_3$ | $NO_2$ | |
| 1.375 | 5-Cl, 7-$CF_3$ | H | $NO_2$ | $CF_3$ | |
| 1.376 | 5,7-$Cl_2$ | Cl | $CF_3$ | $NO_2$ | |
| 1.377 | 5,7-$Cl_2$ | H | $NO_2$ | $CF_3$ | |
| 1.378 | 5,7-$Br_2$ | Cl | $CF_3$ | $NO_2$ | |
| 1.379 | 5,7-$Br_2$ | H | $NO_2$ | $CF_3$ | |
| 1.380 | 5-CN, 7-Cl | Cl | $CF_3$ | $NO_2$ | |
| 1.381 | 5-CN, 7-Cl | H | $NO_2$ | $CF_3$ | |
| 1.382 | 5-CN, 4-Cl | Cl | $CF_3$ | $NO_2$ | |
| 1.383 | 5-CN, 4-Cl | H | $NO_2$ | $CF_3$ | |

Tabelle 2

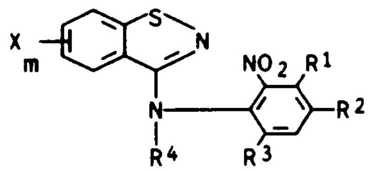

I,

| Nr. | $X_m$ | $R^1$ | $R^2$ | $R^3$ | $R^4$ | Schmp. (°C) |
|---|---|---|---|---|---|---|
| 2.1 | - | Cl | $CF_3$ | $NO_2$ | $SO_2$Phenyl | |
| 2.2 | - | H | $NO_2$ | $CF_3$ | $SO_2Ph$ | |
| 2.3 | - | H | $CF_3$ | H | $SO_2Ph$ | |
| 2.4 | - | H | $CF_3$ | H | $COCH_3$ | 108-111 |
| 2.5 | - | H | $CF_3$ | H | $COOCH_3$ | |
| 2.6 | 5-Cl | Cl | $CF_3$ | $NO_2$ | $SO_2Ph$ | |
| 2.7 | 5-Cl | H | $NO_2$ | $CF_3$ | $SO_2Ph$ | |
| 2.8 | 5-$NO_2$, 7-Cl | Cl | $CF_3$ | $NO_2$ | $SO_2Ph$ | |
| 2.9 | 5-$NO_2$, 7-Cl | Cl | $CF_3$ | $NO_2$ | $CONH_2$ | |
| 2.10 | 5-$NO_2$, 7-Cl | H | $NO_2$ | $CF_3$ | $COOCH_2Ph$ | |
| 2.11 | 5-$NO_2$, 7-Cl | H | $NO_2$ | $CF_3$ | $SO_2Ph$ | |
| 2.12 | 5-$NO_2$, 7-Cl | H | $CF_3$ | H | $SO_2Ph$ | |
| 2.13 | 5-$NO_2$ | H | $CF_3$ | H | $COCH_2Cl$ | |
| 2.14 | 5-$NO_2$ | H | $CF_3$ | H | $SO_2Ph$ | |
| 2.15 | 5-$NO_2$ | H | $CF_3$ | H | $COCHCl_2$ | |
| 2.16 | 5-$NO_2$ | Cl | $CF_3$ | $NO_2$ | $SO_2Ph$ | |
| 2.17 | 5-$NO_2$ | Cl | $CF_3$ | $NO_2$ | $COCH_3$ | |
| 2.18 | 5-$NO_2$ | Cl | $CF_3$ | $NO_2$ | $COCHCl_2$ | |
| 2.19 | 5-$NO_2$ | Cl | $CF_3$ | $NO_2$ | COPh | |
| 2.20 | 5-$NO_2$ | Cl | $CF_3$ | $NO_2$ | $COOCH_3$ | |
| 2.21 | 5-$NO_2$ | Cl | $CF_3$ | $NO_2$ | $CONH_2$ | |
| 2.22 | 5-$NO_2$ | Cl | $CF_3$ | $NO_2$ | CHO | |
| 2.23 | 5,7-$Cl_2$ | Cl | $CF_3$ | $NO_2$ | $SO_2Ph$ | |
| 2.24 | 5,7-$Cl_2$ | H | $NO_2$ | $CF_3$ | $SO_2Ph$ | |
| 2.25 | 5,7-$Cl_2$ | H | $NO_2$ | $CF_3$ | $COOCH_3$ | |
| 2.26 | 5,7-$Cl_2$ | H | $NO_2$ | $CF_3$ | $COCH_2Cl$ | |
| 2.27 | 4-Cl | Cl | $CF_3$ | $NO_2$ | $SO_2Ph$ | |
| 2.28 | 4-Cl | H | $NO_2$ | $CF_3$ | $SO_2Ph$ | |
| 2.29 | 6-$NO_2$ | Cl | $CF_3$ | $NO_2$ | $SO_2Ph$ | |
| 2.30 | 6-$NO_2$ | H | $NO_2$ | $CF_3$ | $SO_2Ph$ | |

Tabelle 2 (Fortsetzung)

| Nr. | $X_m$ | $R^1$ | $R^2$ | $R^3$ | $R^4$ | Schmp. (°C) |
|---|---|---|---|---|---|---|
| 2.31 | 6-$CF_3$ | Cl | $CF_3$ | $NO_2$ | $SO_2Ph$ | |
| 2.32 | 6-$CF_3$ | H | $NO_2$ | $CF_3$ | $SO_2Ph$ | |
| 2.33 | 5-$CF_3$ | Cl | $CF_3$ | $NO_2$ | $COCH_2Cl$ | |
| 2.34 | 5-$CF_3$ | Cl | $CF_3$ | $NO_2$ | $SO_2Ph$ | |
| 2.35 | 5-$CF_3$ | Cl | $CF_3$ | $NO_2$ | $COOCH_3$ | |
| 2.36 | 5-$CF_3$ | Cl | $CF_3$ | $NO_2$ | $CONH_2$ | |
| 2.37 | 5-$CF_3$ | Cl | $CF_3$ | $NO_2$ | COPh | |
| 2.38 | 5-$CF_3$ | H | $CF_3$ | H | $SO_2Ph$ | |
| 2.39 | 5-$CF_3$ | H | $NO_2$ | $CF_3$ | $SO_2Ph$ | |
| 2.40 | 5-$CF_3$ | H | $NO_2$ | $CF_3$ | $COOCH_3$ | |
| 2.41 | 5-$CF_3$ | H | $NO_2$ | $CF_3$ | $CONH_2$ | |
| 2.42 | 5-$CF_3$ | H | $NO_2$ | $CF_3$ | COPh | |
| 2.43 | 5-$CF_3$ | H | $NO_2$ | $CF_3$ | $COCHCl_2$ | |
| 2.44 | 4-$OCH_3$ | Cl | $CF_3$ | $NO_2$ | $SO_2Ph$ | |
| 2.45 | 7-$NO_2$ | Cl | $CF_3$ | $NO_2$ | $SO_2Ph$ | |
| 2.46 | 7-$NO_2$ | H | $NO_2$ | $CF_3$ | $SO_2Ph$ | |

Tabelle 3

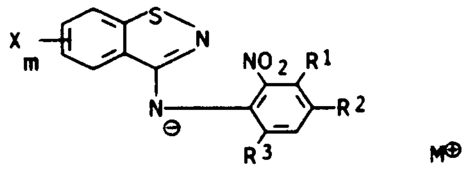

| Nr. | $X_m$ | $R^1$ | $R^2$ | $R^3$ | $M^{\oplus}$ | Schmp. (°C) |
|---|---|---|---|---|---|---|
| 3.1 | - | Cl | $CF_3$ | $NO_2$ | $K^{\oplus}$ | |
| 3.2 | - | Cl | $CF_3$ | $NO_2$ | $N(C_4H_9)_4^{\oplus}$ | 165 |
| 3.3 | - | H | $NO_2$ | $CF_3$ | $N(C_4H_9)_4^{\oplus}$ | |
| 3.4 | - | H | $CF_3$ | H | $N(C_4H_9)_4^{\oplus}$ | |
| 3.5 | 5-$NO_2$ | Cl | $CF_3$ | $NO_2$ | $K^{\oplus}$ | 185-190 |
| 3.6 | 5-$NO_2$ | Cl | $CF_3$ | $NO_2$ | $N(CH_3)_3CH_2C_6H_5^{\oplus}$ | |
| 3.7 | 5-$NO_2$ | Cl | $CF_3$ | $NO_2$ | $N(C_4H_9)_4^{\oplus}$ | 143 |
| 3.8 | 5-$NO_2$ | H | $CF_3$ | H | $N(C_4H_9)_4^{\oplus}$ | |
| 3.9 | 5-$NO_2$ | H | $CF_3$ | $NO_2$ | $N(C_4H_9)_4^{\oplus}$ | 159 |
| 3.10 | 5-$NO_2$ | H | $NO_2$ | $CF_3$ | $N(C_4H_9)_4^{\oplus}$ | |
| 3.11 | 5-$NO_2$ | Cl | $COOC_2H_5$ | $NO_2$ | $N(C_4H_9)_4^{\oplus}$ | 159-161 |
| 3.12 | 5-$NO_2$ | H | $COOCH_3$ | $NO_2$ | $N(C_4H_9)_4^{\oplus}$ | 93 |
| 3.13 | 5-$NO_2$ | H | $SO_2CH_3$ | $NO_2$ | $N(C_4H_9)_4^{\oplus}$ | 142-145 |
| 3.14 | 5-$NO_2$ | Cl | Cl | Cl | $N(C_4H_9)_4^{\oplus}$ | 105-110 |
| 3.15 | 5-Cl | Cl | $CF_3$ | $NO_2$ | $K^{\oplus}$ | |
| 3.16 | 5-Cl | Cl | $CF_3$ | $NO_2$ | $N(C_4H_9)_4^{\oplus}$ | |
| 3.17 | 5-Cl | H | $NO_2$ | $CF_3$ | $N(C_4H_9)_4^{\oplus}$ | |
| 3.18 | 5-$CF_3$ | Cl | $CF_3$ | NO2 | $K^{\oplus}$ | |
| 3.19 | 5-$CF_3$ | Cl | $CF_3$ | $NO_2$ | $N(CH_3)_4^{\oplus}$ | |
| 3.20 | 5-$CF_3$ | Cl | $CF_3$ | $NO_2$ | $N(C_4H_9)_4^{\oplus}$ | |
| 3.21 | 5-$CF_3$ | Cl | $CF_3$ | $NO_2$ | $NH_2(i\text{-}C_3H_7)_2^{\oplus}$ | |
| 3.22 | 5-$CF_3$ | Cl | $CF_3$ | $NO_2$ | $(CH_3)_3NCH_2CH_2OH^{\oplus}$ | |
| 3.23 | 5-$CF_3$ | H | $NO_2$ | $CF_3$ | $K^{\oplus}$ | |
| 3.24 | 5-$CF_3$ | H | $NO_2$ | $CF_3$ | $N(C_4H_9)_4^{\oplus}$ | |
| 3.25 | 5-$CF_3$ | H | $NO_2$ | $CF_3$ | $NH_2(i\text{-}C_3H_7)_2^{\oplus}$ | |
| 3.26 | 5-Br | Cl | $CF_3$ | $NO_2$ | $N(C_4H_9)_4^{\oplus}$ | |
| 3.27 | 5-Br | H | $NO_2$ | $CF_3$ | $N(C_4H_9)_4^{\oplus}$ | |
| 3.28 | 5,7-$Cl_2$ | Cl | $CF_3$ | $NO_2$ | $K^{\oplus}$ | |
| 3.29 | 5,7-$Cl_2$ | Cl | $CF_3$ | $NO_2$ | $N(C_4H_9)_4^{\oplus}$ | |

Tabelle 3 (Fortsetzung)

| Nr. | $X_m$ | $R^1$ | $R^2$ | $R^3$ | $M^+$ | Schmp. (°C) |
|---|---|---|---|---|---|---|
| 3.30 | 5,7-$Cl_2$ | H | $NO_2$ | $CF_3$ | $K^{\oplus}$ | |
| 3.31 | 5,7-$Cl_2$ | H | $NO_2$ | $CF_3$ | $N(C_4H_9)_4^{\oplus}$ | |
| 3.32 | 5-$NO_2$, 7-Cl | Cl | $CF_3$ | $NO_2$ | $N(C_4H_9)_4^{\oplus}$ | |
| 3.33 | 5-$NO_2$, 7-Cl | H | $NO_2$ | $CF_3$ | $N(C_4H_9)_4^{\oplus}$ | |
| 3.34 | 4-Cl | Cl | $CF_3$ | $NO_2$ | $N(C_4H_9)_4^{\oplus}$ | |
| 3.35 | 4-Cl | H | $NO_2$ | $CF_3$ | $N(C_4H_9)_4^{\oplus}$ | |
| 3.36 | 6-$NO_2$ | Cl | $CF_3$ | $NO_2$ | $N(C_4H_9)_4^{\oplus}$ | |
| 3.37 | 6-$NO_2$ | H | $NO_2$ | $CF_3$ | $N(C_4H_9)_4^{\oplus}$ | |
| 3.38 | 6-$CF_3$ | Cl | $CF_3$ | $NO_2$ | $N(C_4H_9)_4^{\oplus}$ | |
| 3.39 | 6-$CF_3$ | H | $NO_2$ | $CF_3$ | $N(C_4H_9)_4^{\oplus}$ | |
| 3.40 | 4-$OCH_3$ | Cl | $CF_3$ | $NO_2$ | $N(C_4H_9)_4^{\oplus}$ | |
| 3.41 | 7-$CF_3$ | Cl | $CF_3$ | $NO_2$ | $N(C_4H_9)_4^{\oplus}$ | |
| 3.42 | 7-$NO_2$ | Cl | $CF_3$ | $NO_2$ | $N(C_4H_9)_4^{\oplus}$ | |
| 3.43 | 7-$NO_2$ | H | $NO_2$ | $CF_3$ | $N(C_4H_9)_4^{\oplus}$ | |
| 3.44 | 7-Cl | Cl | $CF_3$ | $NO_2$ | $N(C_4H_9)_4^{\oplus}$ | |
| 3.45 | 7-Cl | H | $NO_2$ | $CF_3$ | $N(C_4H_9)_4^{\oplus}$ | |
| 3.46 | 7-Cl | H | $CH_3$ | $NO_2$ | $N(C_4H_9)_4^{\oplus}$ | |
| 3.47 | 6-Cl | Cl | $CF_3$ | $NO_2$ | $N(C_4H_9)_4^{\oplus}$ | |
| 3.48 | 6-Cl | H | $NO_2$ | $CF_3$ | $N(C_4H_9)_4^{\oplus}$ | |
| 3.49 | 6-Cl | Cl | $CF_3$ | $NO_2$ | $K^{\oplus}$ | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltarn) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,

Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Materialien mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Zur Saatgutbehandlung werden im allgemeinen Wirkstoffmengen von 0,001 bis 50 g, vorzugsweise 0,01 bis 10 g je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1.1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 1.16 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 1.33 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1.46 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1.82 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1.1 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1.16 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1.33 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1.46 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäureharnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstrumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoffe wurden 2-(2,6-Dinitro-3-chlor-4-trifluormethyl-anilino)-6-methoxybenzthiazol (A) und 2-(2,6-Dinitro-3-chlor-4-trifluormethyl-anilino)-6-nitrobenzthiazol (B) - bekannt aus EP 244 705 - benutzt.

Anwendungsbeispiel 1

Wirksamkeit gegen Botrytis cinerea

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22-24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten. Es wurde der Befall der Blätter geprüft.

Das Ergebnis zeigt, daß der Wirkstoff 1.46 bei der Anwendung als 0,05 %ige (Gew.-%) Spritzbrühe eine bessere fungizide Wirkung zeigt (97 %) als die bekannten Vergleichswirkstoffe A (30 %) und B (20 %).

Anwendungsbeispiel 2

Wirksamkeit gegen Rebenperonospora

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) inoculiert. Danach wurden die Reben zunächst für 48 Stunden in einer wasserdampfgesättigten Kammer bei 24 °C und anschließend für 5 Tage in einem Gewächshaus mit Temperaturen zwischen 20 und 30 °C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 1.1, 1.16, 1.33, 1.46 und 1.82 bei der Anwendung als 0,05 %ige Spritzbrühe eine bessere fungizide Wirkung zeigen (100 %) als die bekannten Vergleichswirkstoffe A (65 %) und B (65 %).

Anwendungsbeispiel 3

Wirksamkeit gegen Phytophthora infestans

Blätter von Topfpflanzen der Sorte "Große Fleischtomate" wurden mit wäßriger Spritzbrühe, die 80 % Wirkstoff und 20 % Emulgiermittel in der Trockensubstanz enthielt, besprüht. Nach 24 Stunden wurden die Blätter mit einer Zoosporenaufschwemmung des Pilzes Phytophthora infestans infiziert. Die Pflanzen wurden dann in einer wasserdampfgesättigten Kammer bei Temperaturen zwischen 16 und 18 °C aufgestellt. Nach 6 Tagen hatte sich die Krankheit auf den unbehandelten, jedoch infizierten Kontrollpflanzen so stark entwickelt, daß die fungizide Wirksamkeit der Substanzen beurteilt werden konnte.

Das Ergebnis zeigt, daß die Wirkstoffe 1.16, 1.33, 1.46 und 1.82 bei der Anwendung als 0,025 %ige Spritzbrühe eine bessere fungizide Wirkung zeigen (97 %) als die bekannten Vergleichswirkstoffe A (20 %) und B (50 %).

## Patentansprüche

1. 3-Anilino-benzisothiazole der Formel I

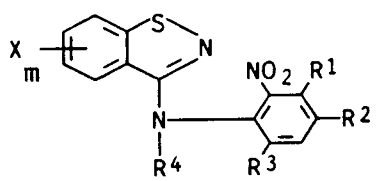

I,

in welcher

| | |
|---|---|
| X | Wasserstoff, $NO_2$, CN, Halogen, $SO_2$-$C_1$-$C_4$-Alkyl, $C_1$-$C_4$-Alkyl, $C_3$-$C_6$-Cycloalkyl, $C_1$-$C_4$-Halogenalkyl, $C_1$-$C_4$-Alkoxy, $C_1$-$C_4$-Halogenalkoxy, $C_1$-$C_4$-Alkylthio, gegebenenfalls im Phenylrest durch $C_1$-$C_4$-Alkyl, Halogen, Nitro substituiertes Phenoxy oder Phenylthio bedeutet, |
| m | eine ganze Zahl von 1 bis 4 ist, wobei die einzelnen Reste gleich oder verschieden sind, wenn m größer als 1 ist, |
| $R^1$ | Wasserstoff, Halogen, $C_1$-$C_4$-Alkoxy, $C_1$-$C_4$-Halogenalkyl, $C_1$-$C_4$-Alkylthio, $C_1$-$C_4$-Halogenalkoxy, gegebenenfalls im Phenylrest durch $C_1$-$C_4$-Alkyl, Halogen, Nitro substituiertes Phenoxy, Phenylthio, Benzyloxy oder Benzylthio bedeutet, |
| $R^2$, $R^3$ | unabhängig voneinander Wasserstoff, $NO_2$, Halogen, CN, $C_1$-$C_6$-Alkyl, $SO_2$-$C_1$-$C_4$-Alkyl, $C_1$-$C_6$-Halogenalkyl, $SO_2NR^5R^6$, $C_1$-$C_4$-Halogenalkoxy, $COOR^5$, $CONR^5R^6$, |
| $R^4$ | Wasserstoff, $COOR^7$, $CONR^5R^6$, CHO, $COR^7$, $SO_2R^7$, |
| $R^5$, $R^6$ | unabhängig voneinander Wasserstoff, $C_1$-$C_4$-Alkyl, |
| $R^7$ | $C_1$-$C_4$-Alkyl, $C_1$-$C_4$-Halogenalkyl, Benzyl, gegebenenfalls durch $C_1$-$C_4$-Alkyl, Halogen, Nitro substituiertes Phenyl oder Naphthyl bedeutet |

und ihre pflanzenverträgliche Salze.

2. Verfahren zur Herstellung der Benzisothiazole der Formel I gemäß Anspruch 1, in der $R^4$ Wasserstoff bedeutet, dadurch gekennzeichnet, daß man ein Benzisothiazol der Formel II, in der X und m die in Anspruch 1 angegebenen Bedeutungen haben, mit einer Phenylverbindung der Formel III,

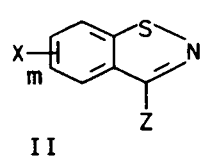

II

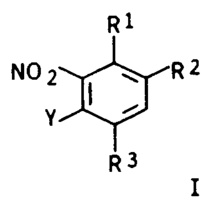

III

in der $R^1$, $R^2$, $R^3$ die in Anspruch 1 angegebenen Bedeutungen haben, gegebenenfalls in Anwesenheit einer organischen oder anorganischen Base umsetzt, wobei Y und Z für $NH_2$ oder Halogen stehen und Y und Z jeweils voneinander verschieden sind.

3. Verfahren zur Herstellung der Benzisothiazole der Formel I gemäß Anspruch 1, in der $R^4$ verschieden von Wasserstoff ist, dadurch gekennzeichnet, daß man ein Benzisothiazol der Formel I, in der $R^4$ Wasserstoff ist und X, m, $R^1$, $R^2$, $R^3$, $R^5$, $R^6$, $R^7$, die in Anspruch 1 angegebenen Bedeutungen haben, mit einem Acylierungs- oder Sulfonylierungsmittel der Formel IV

$LR^4$ IV,

in der $R^4$ die in Anspruch 1 angegebene Bedeutung außer Wasserstoff hat und L für eine nucleophil verdrängbare Abgangsgruppe steht, gegebenenfalls in Gegenwart einer organischen oder anorganischen Base umsetzt.

**4.** Fungizides Mittel, enthaltend einen festen oder flüssigen Trägerstoff und eine fungizid wirksame Menge einer Verbindung der Formel I gemäß Anspruch 1.

**5.** Verfahren zur Herstellung von Fungiziden, dadurch gekennzeichnet, daß man eine Verbindung der Formel I gemäß Anspruch 1 mit einem festen oder flüssigen Trägerstoff mischt.

**6.** Verfahren zur Bekämpfung von Pilzen, dadurch gekennzeichnet, daß man eine fungizid wirksame Menge einer Verbindung der Formel I gemäß Anspruch 1 auf die Pilze oder auf durch Pilzbefall bedrohte Pflanzen, Saatgüter, Materialien oder den Erdboden einwirken läßt.

**7.** Verbindung der Formel I gemäß Anspruch 1, dadurch gekennzeichnet, daß $X_m$ 5-$NO_2$, $R^1$ Cl, $R^2$ $CF_3$, $R^3$ $NO_2$ und $R^4$ Wasserstoff bedeutet.

**8.** Verbindung der Formel I gemäß Anspruch 1, dadurch gekennzeichnet, daß $X_m$ Wasserstoff, $R^1$ Cl, $R^2$ $CF_3$, $R^3$ $NO_2$ und $R^4$ Wasserstoff bedeutet.

**9.** Verbindung der Formel I gemäß Anspruch 1, dadurch gekennzeichnet, daß $X_m$ Wasserstoff, $R^1$ Wasserstoff, $R^2$ $NO_2$, $R^3$ $CF_3$ und $R^4$ Wasserstoff bedeutet.

## Claims

**1.** A 3-anilinobenzisothiazole of the formula I

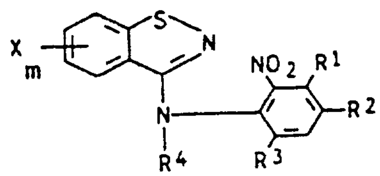

I,

where

| | |
|---|---|
| X | is hydrogen, $NO_2$, CN, halogen, $SO_2$-$C_1$-$C_4$-alkyl, $C_1$-$C_4$-alkyl, $C_3$-$C_6$-cycloalkyl, $C_1$-$C_4$-haloalkyl, $C_1$-$C_4$-alkoxy, $C_1$-$C_4$-haloalkoxy, $C_1$-$C_4$-alkylthio, or phenoxy or phenylthio, each of which is unsubstituted or substituted in the phenyl radical by $C_1$-$C_4$-alkyl, halogen or nitro, |
| m | is an integer from 1 to 4, the individual radicals being identical or different if m is greater than 1, |
| $R^1$ | is hydrogen, halogen, $C_1$-$C_4$-alkoxy, $C_1$-$C_4$-haloalkyl, $C_1$-$C_4$-alkylthio, $C_1$-$C_4$-haloalkoxy, or phenoxy, phenylthio, benzyloxy or benzylthio, each of which is unsubstituted or substituted in the phenyl radical by $C_1$-$C_4$-alkyl, halogen or nitro, |
| $R^2$ and $R^3$ | independently of one another are hydrogen, $NO_2$, halogen, CN, $C_1$-$C_6$-alkyl, $SO_2$-$C_1$-$C_4$-alkyl, $C_1$-$C_6$-haloalkyl, $SO_2NR^5R^6$, $C_1$-$C_4$-haloalkoxy, $COOR^5$ or $CONR^5R^6$, |
| $R^4$ | is hydrogen, $COOR^7$, $CONR^5R^6$, CHO, $COR^7$ or $SO_2R^7$, |
| $R^5$ and $R^6$ | independently of one another are hydrogen or $C_1$-$C_4$-alkyl, |
| $R^7$ | is $C_1$-$C_4$-alkyl, $C_1$-$C_4$-haloalkyl, benzyl, or phenyl or naphthyl, each of which is unsubstituted or substituted by $C_1$-$C_4$-alkyl, halogen or nitro, |

or its plant-tolerable salts.

**2.** A process for preparing the benzisothiazoles of the formula I as claimed in claim 1 where $R^4$ is hydrogen, which comprises reacting a benzisothiazole of the formula II where X and m have the meanings indicated in claim 1, with a phenyl compound of the formula III

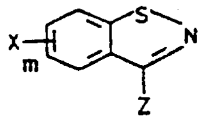

II

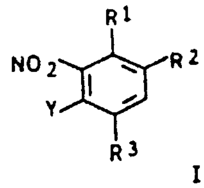

III

where $R^1$, $R^2$ and $R^3$ have the meanings indicated in claim 1, in the presence of absence of an organic or inorganic base, Y and Z being $NH_2$ or halogen and Y and Z in each case being different from one another.

**3.** A process for preparing the benzisothiazoles of the formula I as claimed in claim 1 where $R^4$ is different from hydrogen, which comprises reacting a benzisothiazole of the formula I where $R^4$ is hydrogen and X, m, $R^1$, $R^2$, $R^3$, $R^5$, $R^6$ and $R^7$ have the meanings indicated in claim 1, with an acylating or sulphonylating agent of the formula IV

$LR^4$ IV,

where $R^4$ has the meanings indicated in claim 1 apart from hydrogen and L is a nucleophilically displaceable leaving group, in the presence or absence of an organic or inorganic base.

**4.** A fungicidal composition containing a solid or liquid carrier and a fungicidally effective amount of a compound of the formula I as claimed in claim 1.

**5.** A process for preparing fungicides, which comprises mixing a compound of the formula I as claimed in claim 1 with a solid or liquid carrier.

**6.** A process for controlling fungi, which comprises allowing a fungicidally effective amount of a compound of the formula I as claimed in claim 1 to act on the fungi or on plants, seeds, materials or the soil threatened by fungal attack.

**7.** A compound of the formula I as claimed in claim 1, wherein $X_m$ is 5-$NO_2$, $R^1$ is Cl, $R^2$ is $CF_3$, $R^3$ is $NO_2$ and $R^4$ is hydrogen.

**8.** A compound of the formula I as claimed in claim 1, wherein $X_m$ is hydrogen, $R^1$ is Cl, $R^2$ is $CF_3$, $R^3$ is $NO_2$ and $R^4$ is hydrogen.

**9.** A compound of the formula I as claimed in claim 1, wherein $X_m$ is hydrogen, $R^1$ is hydrogen, $R^2$ is $NO_2$, $R^3$ is $CF_3$ and $R^4$ is hydrogen.

**Revendications**

**1.** 3-anilino-benzisothiazoles de la formule I

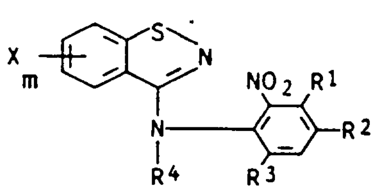

I,

dans laquelle
X représente hydrogène $NO_2$, CN, halogène, $SO_2$-alkyle en C1-C4, alkyle en C1-C4, cycloalkyle en C3-C6, halogénalkyle en C1-C4, alcoxy en C1-C4, halogénalcoxy en C1-C4, alkylthio en C1-C4, phénoxy ou phénylthio, éventuellement substitué dans le reste phényle par alkyle en C1-C4, halogène, nitro
m est un nombre entier de 1 à 4, les restes étant identiques ou différents, quand m est supérieur à 1

R1 représente hydrogène, halogène, alcoxy en C1-C4, halogénalkyle en C1-C4, alkylthio en C1-C4, halogénalcoxy en C1-C4, phénoxy éventuellement substitué dans le reste phényle par alkyle en C1-C4, halogène nitro, phénylthio, benzyloxy ou benzylthio

R2, R3, représentent indépendamment l'un de l'autre, hydrogène, $NO_2$, halogène, CN, alkyle en C1-C6, $SO_2$-alkyle en C1-C4, halogénalkyle en C1-C6, $SO_2NR^5R^6$, halogénalcoxy en C1-C4, $COOR^5$, $CONR^5R^6$

R4, hydrogène, $COOR^7$, $CONR^5R^6$, CHO, $COR^7$, $SO_2R^7$

R5, R6, indépendamment l'un de l'autre, hydrogène, alkyle en C1-C4

R7 représente alkyle en C1-C4, halogénalkyle en C1-C4, benzyle, phényle ou naphtyle éventuellement substitué par alkyle en C1-C4, halogène, nitro

et leurs sels acceptables par les plantes.

**2.** Procédé de préparation de benzisothiazoles de formule I, selon la revendication 1 dans laquelle $R^4$ représente l'hydrogène, caractérisé par le fait qu'on fait réagir, éventuellement en présence d'une base organique ou inorganique, un benzisothiazole de formule II, dans laquelle X et m ont les significations données dans la revendication 1, avec un composé de phényle de formule III

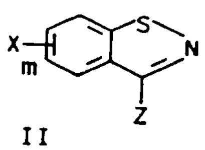

II

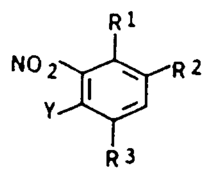

III

dans laquelle $R^1$, $R^2$, $R^3$ ont les significations données dans la revendication 1
Y et Z étant mis pour $NH_2$ ou halogène et Y et Z étant différents l'un de l'autre.

**3.** Procédé de préparation de benzisothiazoles de formule I, selon la revendication 1 dans laquelle $R^4$ est différent de hydrogène, caractérisé par le fait que on fait réagir, éventuellement en présence d'une base organique ou inorganique, un benzisothiazole de formule I dans laquelle $R^4$ est l'hydrogène et X, m, $R^1$, $R^2$, $R^3$, $R^5$, $R^6$, $R^7$ ont les significations données dans le revendication 1, avec un agent d'acylation ou sulfonylation de formule IV

$LR^4$ IV

dans laquelle $R^4$ a la signification indiquée dans la revendication 1, à l'exception d'hydrogène et L est mis pour un groupe éliminable déplaçable nucléophile.

**4.** Agent fongicide contenant un support solide ou liquide et une quantité à activité fongicide d'un composé de formule I selon la revendication 1.

**5.** Procédé de préparation de fongicides, caractérisé par le fait qu'on mélange un composé de formule I selon la revendication 1 avec un support solide ou liquide.

**6.** Procédé de lutte contre les champignons, caractérisé par le fait qu'on fait agir sur les champignons ou sur les plantes, semences, matériaux ou sol menacés d'une attaque par les champignons, une quantité à activité fongicide d'un composé de formule I selon la revendication 1.

**7.** Composé de formule I selon la revendication 1, caractérisé par le fait que $X_m$ représente 5-$NO_2$, $R^1Cl$, $R^2CF_3$, $R^3NO_2$ et $R^4$ hydrogène.

**8.** Composé de formule I selon la revendication 1, caractérisé par le fait que $X_m$ représente hydrogène, $R^1Cl$, $R^2CF_3$, $R^3NO_2$ et $R^4$ hydrogène.

**9.** Composé de formule I selon la revendication 1, caractérisé par le fait que $X_m$ représente hydrogène, $R^1$ hydrogène, $R^2NO_2$, $R^3CF_3$ et $R^4$ hydrogène.